# EUROPEAN PATENT SPECIFICATION

(11) **EP 3 344 765 B1**
(45) Date of publication and mention of the grant of the patent: **19.12.2018**
(21) Application number: 16757872.3
(22) Date of filing: 25.08.2016
(51) Int. Cl.: C12N 9/96, C11D 3/386

(54) **LIQUID DETERGENCY COMPOSITION COMPRISING LIPASE AND PROTEASE**
FLÜSSIGE WASCHMITTELZUSAMMENSETZUNG MIT LIPASE UND PROTEASE
COMPOSITION DÉTERGENTE LIQUIDE COMPRENANT UNE LIPASE ET UNE PROTÉASE

(30) Priority: 28.08.2015 EP 15182934; 28.08.2015 EP 15182936; 28.08.2015 EP 15182937
(43) Date of publication of application: 11.07.2018
(73) Proprietor: Unilever NV, 3013 AL Rotterdam (NL); Unilever PLC, London, Greater London EC4Y 0DY (GB)
(72) Inventor: DE ROSE, Simone, Antonio, Exeter Devon EX4 4QD (GB); DOWD, Andrew, Wirral Merseyside CH63 3JW (GB); LANG, Dietmar, Andreas, Wirral Merseyside CH63 3JW (GB); LITTLECHILD-BOND, Jennifer, Ann, Exeter Devon EX4 4QD (GB); NOVAK, Halina, Rose, 9820 Merelbeke (BE); PARRY, Neil, James, Wirral Merseyside CH63 3JW (GB); SINGH, Sukriti, Wirral Merseyside CH63 3JW (GB)
(74) Representative: McHugh, Paul Edward
(86) International application number: PCT/EP2016/070095
(87) International publication number: WO 2017/036917

(56) References cited:
- EP-A1- 1 088 887
- WO-A1-02/061067
- WO-A1-2005/080561
- WO-A2-2006/046865
- LOPEZ-SERRANO P ET AL: "CROSS-LINKED ENZYME AGGREGATES WITH ENHANCED ACTIVITY: APPLICATION TO LIPASES", BIOTECHNOLOGY LETTERS, SPRINGER NETHERLANDS, NL, vol. 24, no. 16, 1 August 2002 (2002-08-01), pages 1379-1383, XP009003775, ISSN: 0141-5492, DOI: 10.1023/A:1019863314646

## Description

Liquid detergency compositions comprising enzymes have become more prevalent over the last few years. In particular liquid detergency products comprising lipases and proteases have found use for more effective removal of fat- and protein-based stains.

However, a major problem in using such enzymes in liquid detergency compositions is that they are prone to decrease in enzyme activity over the life time of the liquid detergency composition, leading to reduced stain removal efficiency. A further problem in formulating multi-enzyme liquid detergency compositions comprising protease is the tendency of the protease to inactivate other enzymes in the composition by proteolytic attack. One way of increasing enzyme activity is to simply add more enzyme to the detergency composition, but this leads to cost increase. Indeed the detergency composition market although being a high volume market tends to have low profit margin due to ingredient costs.

Another way of maintaining enzyme activity over the shelf-life of a detergency composition is by use of crystallized enzymes. Such crystallized enzymes are described in US2002/0137156, US2002/0082181 and US2001/0046493. However, crystallized enzymes are more expensive than their non-crystallized counterparts and also their manufacture itself is a time-consuming and laborious process. As such, use of crystallized enzymes to maintain the stability and activity of enzymes in a liquid detergency composition is not a useful technology.

Alternatively, binding enzymes to a scaffold, such as an activated polymer (US6,030,933) or non-material surface (US2007/077565) or activated substrate (US2004/0029242) has also been described. Again, use of such scaffolds and the additional step of binding the enzymes thereto increases costs and enzyme-preparation complexity. Therefore these are also considered not useful technologies to prepare liquid detergency compositions comprising enzymes.

US2008/0296231 discloses a method for the preparation of cross-linked enzyme aggregates (CLEAs) that allows use of a wider range of reagents and the possibility to obtain enzyme aggregates with improved properties (US2008/0296231). Cross-linked enzyme aggregates containing lipase are commercially available (e.g. from Sigma Aldrich or Novozymes).

It is an object of the present invention to provide a simple and cost-effective liquid detergency composition, preferably a liquid laundry composition, comprising lipase and protease, wherein the lipase maintains improved stability and activity during storage conditions.

### Summary of the invention

One or more of the above objectives have been met by a liquid detergency composition comprising a specific lipase, which was found to more stable during storage even in the presence of protease.

Therefore, in a first aspect the invention relates to a liquid detergency composition comprising:
- a protease, and
- a lipase, wherein the lipase comprises a polypeptide having an amino acid sequence which has at least 90 percent sequence identity with the wild-type lipase derived from *Humicola lanuginosa* strain DSM 4109 and, compared to said wild-type lipase, comprises a substitution of an electrically neutral or negatively charged amino acid within 15 A of E1 or Q249 with a positively charged amino acid.

Said liquid detergency composition was found to exhibit superior enzyme stability and activity of the lipase when compared with use of other commercially available lipases (e.g. *Thermomyces lanuginose* Lipase). This was found to be the case even when cross-linked aggregates of the lipase was used in comparison with commercially available cross-linked enzyme aggregates of lipase (*Thermomyces lanuginose* Lipase Sigma cat # 07676, Supplier: Sigma Aldrich).

It is believed that the improved resistance to proteolytic attack is further enhanced by a specific method to manufacture the CLEAs of lipase. Indeed enzymes have complex three dimensional structures and several functional groups by with they may be cross-linked to form CLEAs. For example cross-linking may be based on cross-linking using primary amines (-NH2), carboxyls (-COOH), sufhydryls (-SH) and/or carbonyls (-CHO). Different cross-linking agents may have different reactivity and selectivity, in particular to cross-link amino-acids within the three dimensional structure. It was found that cross-linking by use of a di-aldehyde in the context for lipases is particularly advantageous to maintain stability and activity of CLEA lipases.

Therefore, a process for the manufacture of the CLEA of lipase comprises the following steps:
1) providing an aqueous suspension of the lipase;
2) precipitation of the enzyme;
3) Cross-linking the enzyme by addition of a di-aldehyde.

### Detailed description

All percentages mentioned herein are by weight calculated on the total composition, unless specified otherwise. The abbreviation 'wt.%' is to be understood as % by weight of the total composition unless otherwise specified. It will be appreciated that the total amount of ingredients in the detergency composition will not exceed 100 wt.%.

### Lipase

The liquid detergency composition according to the invention comprises a lipase which comprise a polypeptide having an amino acid sequence which has at least 90 percent sequence identity with the wild-type lipase derived from *Humicola lanuginosa* strain DSM 4109 and compared to said wild-type lipase, comprises a substitution of an electrically neutral or negatively charged amino acid within 15 A of E1 or Q249 with a positively charged amino acid. The lipase preferably further comprises one or more of the following:
(I) a peptide addition at the C-terminal;
(II) a peptide addition at the N-terminal;
(III) the following limitations:
   i. comprises a negatively charged amino acid in position E210 of said wild-type lipase;
   ii comprises a negatively charged amino acid in the region corresponding to positions 90-101 of said wild-type lipase; and
   iii. comprises a neutral or negatively charged amino acid at a position corresponding to N94 of said wild-type lipase; and/or
   iv. has a negative charge or neutral charge in the region corresponding to positions 90-101 of said wild-type lipase.

These are available under the Lipex™ brand from Novozymes.

Preferably the amount of lipase according to the invention is from 0.01 to 6 wt.%, more preferably from 0.1 to 5 wt.%, even more preferably from 0.2 to 4 wt.%, still even more preferably from 0.5 to 3 wt.% and still even more preferably from 0.7 to 2.0 wt.%.

Preferably at least part of the lipase according to the invention is cross-linked enzyme aggregate of the lipase, more preferably at least 20 wt.%, even more preferably at least 40 wt.%, still even more preferably at least 60 wt.%, still even more preferably at least 80 wt.% is cross-linked enzyme aggregate of the lipase, based on the total amount of lipase, and still even more preferably the lipase essentially consists of cross-linked enzyme aggregate of the lipase.

The liquid detergency composition according to the invention may comprise further lipases, which include lipases from *Humicola* (synonym *Thermomyces*), e.g. from other *H. lanuginosa* (*T. lanuginosus*) strains or from *H. insolens,* a *Pseudomonas* lipase, e.g. from *P. alcaligenes* or *P. pseudoalcaligenes, P. cepacia, P. stutzeri, P. fluorescens, Pseudomonas* sp. strain SD 705 (WO 95/06720 and WO 96/27002), *P*. *wisconsinensis,* a *Bacillus* lipase, e.g. from *B. subtilis* (Dartois et al. (1993), Biochemica et Biophysica Acta, 1131, 253-360), *B. stearothermophilus* (JP 64/744992) or *B*. *pumilus* (WO 91/16422).

Commercially available lipase enzymes include Lipolase™ and Lipolase Ultra™, and the Bacterial enzyme, Lipomax ® ex Genecor. This is a bacterially derived Lipase, of variant M21L of the lipase of *Pseudomonas alcaligenes* as described in WO 94/25578 to Gist-Brocades (M.M.M.J. Cox, H.B.M. Lenting, L.J.S.M. Mulleners and J.M. van der Laan).

The liquid detergency composition according to the invention comprises one or more proteases. Preferred proteases are serine proteases or metallo proteases, more preferably an alkaline microbial protease or a trypsin-like protease. More preferred (commercially available) proteases are Alcalase™, Savinase™, Primase™, Duralase™, Dyrazym™, Esperase™, Everlase™, Polarzyme™, Kannase™ and Coronase™, Relase™, (Novozymes A/S), Maxatase™, Maxacal™, Maxapem™, Properase™, Purafect™, Purafect OxP™, FN2™, and FN3™ (Genencor International Inc.). Especially good results were obtained for liquid detergency compositions wherein the protease is Savinase™, Coronase™ and/or Relase™. Therefore, still even more preferably the liquid detergency composition according to the invention comprises Savinase™, Coronase™, Relase™ or mixtures thereof, and more preferably essentially is Relase™.

Preferably the amount of protease is from 0.01 to 6 wt.%, more preferably from 0.1 to 5 wt.%, even more preferably from 0.2 to 4 wt.%, still even more preferably from 0.5 to 3 wt.% and still even more preferably from 0.7 to 2.0 wt.%.

### Other enzymes

Preferably the liquid detergency composition according to the invention comprises further (i.e. in addition to the lipase and protease) enzymes and more preferably comprises one or more amylase, phospholyase, cutinase, cellulase, peroxidase, oxidase, pectate lyase and mannanase enzymes.

The liquid detergency composition according to the invention preferably comprises one or more amylases. Preferred amylases (alpha and/or beta) include those of bacterial or fungal origin. Chemically modified or protein engineered mutants are included. More preferred amylases include, for example, alpha-amylases obtained from Bacillus, e.g. a special strain of *B*. *licheniformis,* described in more detail in GB 1,296,839, or the Bacillus sp. strains disclosed in WO 95/026397 or WO 00/060060. Even more preferred (commercially available) amylases are Duramyl™, Termamyl™, Termamyl Ultra™, Natalase™, Stainzyme™, Fungamyl™ and BAN™ (Novozymes A/S), Rapidase™ and Purastar™ (from Genencor International Inc.), Stainzyme™ and Resilience ™(Novozymes).

Preferably the amount of amylase is from 0.01 to 6 wt.%, more preferably from 0.1 to 5 wt.%, even more preferably from 0.2 to 4 wt.%, still even more preferably from 0.5 to 3 wt.% and still even more preferably from 0.7 to 2.0 wt.%.

The liquid detergency composition according to the invention preferably comprises one or more phospholipases. Phospholipase are classified as EC 3.1.1.4 and/or EC 3.1.1.32. As used herein, the term phospholipase is an enzyme, which has activity towards phospholipids. Phospholipids, such as lecithin or phosphatidylcholine, consist of glycerol esterified with two fatty acids in an outer (sn-1) and the middle (sn-2) positions and esterified with phosphoric acid in the third position; the phosphoric acid, in turn, may be esterified to an amino-alcohol. Phospholipases are enzymes which participate in the hydrolysis of phospholipids. Several types of phospholipase activity can be distinguished, including phospholipases A1 and A2 which hydrolyze one fatty acyl group (in the sn-1 and sn-2 position, respectively) to form lysophospholipid; and lysophospholipase (or phospholipase B) which can hydrolyze the remaining fatty acyl group in lysophospholipid. Phospholipase C and phospholipase D (phosphodiesterases) release diacyl glycerol or phosphatidic acid respectively.

Preferably the amount of phospholipase is from 0.01 to 6 wt.%, more preferably from 0.1 to 5 wt.%, even more preferably from 0.2 to 4 wt.%, still even more preferably from 0.5 to 3 wt.% and still even more preferably from 0.7 to 2.0 wt.%.

The liquid detergency composition according to the invention preferably comprises one or more cutinases. Cutinases are classified in EC 3.1.1.74. The cutinase used according to the invention may be of any origin. Preferably the cutinases are of microbial origin and more preferably of bacterial, of fungal or of yeast origin.

Preferably the amount of cutinase is from 0.01 to 6 wt.%, more preferably from 0.1 to 5 wt.%, even more preferably from 0.2 to 4 wt.%, still even more preferably from 0.5 to 3 wt.% and still even more preferably from 0.7 to 2.0 wt.%.

The liquid detergency composition according to the invention preferably comprises one or more cellulases. Preferred cellulase include those of bacterial, fungal, insect and/or mammalian origin. Chemically modified or protein engineered mutants are included. More preferred are cellulases from the genera *Bacillus, Pseudomonas, Humicola, Fusarium, Thielavia, Acremonium,* e.g. the fungal cellulases produced from *Humicola insolens, Thielavia terrestris, Myceliophthora thermophila,* and *Fusarium oxysporum* disclosed in US 4,435,307, US 5,648,263, US 5,691,178, US 5,776,757, WO 89/09259, WO 96/029397, and WO 98/012307. Even more preferred (commercially available) cellulases are Celluzyme™, Carezyme™, Endolase™, Renozyme™ (Novozymes A/S), Clazinase™ and Puradax HA™ (Genencor International Inc.), and KAC-500(B)™ (Kao Corporation).

Preferably the amount of cellulase is from 0.01 to 6 wt.%, more preferably from 0.1 to 5 wt.%, even more preferably from 0.2 to 4 wt.%, still even more preferably from 0.5 to 3 wt.% and still even more preferably from 0.7 to 2.0 wt.%.

The liquid detergency composition according to the invention preferably comprises one or more peroxidases/oxidases, preferably these are of bacterial, fungal or mammalian origin and more preferably of bacterial origin. Chemically modified or protein engineered mutants are included. Preferably the peroxidases/oxidases are derived from *Aeromonas sp.*

Preferably the amount of peroxidase/oxidase is from 0.01 to 6 wt.%, more preferably from 0.1 to 5 wt.%, even more preferably from 0.2 to 4 wt.%, still even more preferably from 0.5 to 3 wt.% and still even more preferably from 0.7 to 2.0 wt.%.

The liquid detergency composition according to the invention preferably comprises one or more pectate lyases (also called polygalacturonate lyases). Preferred are pectate lyases that have been derived from bacterial genera such as *Erwinia, Pseudomonas, Klebsiella* and *Xanthomonas, Bacillus.* More preferred are pectate lyases obtained from *Bacillus subtilis* (Nasser et al. (1993) FEBS Letts. 335:319-326), *Bacillus sp. YA-14* (Kim et al. (1994) Biosci. Biotech. Biochem. 58:947-949); *Bacillus pumilus* (Dave and Vaughn (1971) J. Bacteriol. 108:166-174), B. *polymyxa* (Nagel and Vaughn (1961) Arch. Biochem. Biophys. 93:344-352), B. *stearothermophilus* (Karbassi and Vaughn (1980) Can. J. Microbiol. 26:377-384), *Bacillus sp.* (Hasegawa and Nagel (1966) J. Food Sci. 31:838-845), *Bacillus sp. RK9* (Kelly and Fogarty (1978) Can. J. Microbiol. 24:1164-1172), as disclosed in Heffron et al., (1995) Mol. Plant-Microbe Interact. 8: 331-334, Henrissat et al., (1995) Plant Physiol. 107: 963-976, as disclosed in WO 99/27083, WO 99/27084, US6,284,524, WO 02/006442 (in particular as disclosed in the Examples).

Even more preferred are (commercially available) pectate lyases are BIOPREP™ and SCOURZYME™ L from Novozymes A/S, Denmark.

Preferably the amount of pectate lyase is from 0.01 to 6 wt.%, more preferably from 0.1 to 5 wt.%, even more preferably from 0.2 to 4 wt.%, still even more preferably from 0.5 to 3 wt.% and still even more preferably from 0.7 to 2.0 wt.%. The liquid detergency composition according to the invention preferably comprises one or more mannanases (EC 3.2.1.78). Preferred mannanases include mannanases of bacterial and fungal origin. More preferred are mannases derived from filamentous fungus genus Aspergillus, preferably *Aspergillus niger* or *Aspergillus aculeatus* (WO 94/25576); *Trichoderma reseei* (as disclosed in WO 93/24622); Bacillus organisms (e.g. as described in Talbot et al., Appl. Environ. Microbiol. Vol.56, No. 11, pp. 3505-3510 (1990), which describes a beta-mannanase derived from *Bacillus stearothermophilus,* Mendoza et al., World J. Microbiol. Biotech., Vol. 10, No. 5, pp. 551-555 (1994), which describes a beta-mannanase derived from *Bacillus subtilis,* JP-A-03047076 which describes a beta-mannanase derived from Bacillus sp., JP-A-63056289 which describes the production of an alkaline, thermostable beta-mannanase, JP-A-63036775 which describes Bacillus microorganism FERM P-8856 which produces beta-mannanase and beta-mannosidase, JP-A-08051975 which describes a alkaline beta-mannanases from alkalophilic Bacillus sp. AM-001, WO97/11164 which described a purified mannanase from *Bacillus amyloliquefaciens,* WO 91/18974 which describes a hemicellulase such as a glucanase, xylanase or mannanase active). Also preferred are the alkaline family 5 and 26 mannanases derived from *Bacillus agaradhaerens, Bacillus licheniformis, Bacillus halodurans, Bacillus clausii,* Bacillus sp., and *Humicola insolens* (as disclosed in WO 99/64619). More preferred bacterial mannases are those described in WO 99/64619. Even more preferred (commercially available) mannanase is Mannaway™ available from Novozymes A/S Denmark.

Preferably the amount of mannase is from 0.01 to 6 wt.%, more preferably from 0.1 to 5 wt.%, even more preferably from 0.2 to 4 wt.%, still even more preferably from 0.5 to 3 wt.% and still even more preferably from 0.7 to 2.0 wt.%.

Preferably the liquid detergency composition according to the invention is ambient-active.

### CLEA

CLEAs of the lipase according to the invention can be prepared using any suitable technique known in the art, such as described in EP1088887 using a di-aldehyde as cross-linking agent.

A more preferred method of making the CLEA of lipase according to the invention comprises the following steps:
1) Providing an aqueous suspension of the lipase, preferably at room temperature and in a suitable buffer. The buffer preferably is a buffer of MES-NaOH, NaCl and CaCl2 having a pH of 6 to 9. More preferably the buffer has from 20 to 50 mM of MES-NaOH, from 100 to 200 mM of NaCl and/or 0.5 to 2 mM of CaCI2. The enzyme concentration preferably is from 0.3 to 2 µM.
2) Addition of emulsifier, preferably a polysorbate and more preferably Tween 80 (i.e. polyoxyethylene (20) sorbitan. Preferably the concentration of the emulsifier is from 10 to 30 mM. Preferably the mixture is stirred, such as at room temperature, for at least 1 minute. The presence of such an emulsifier helps to bring the lipase into an active configuration, which further improves enzyme activity of the lipase in the final CLEA.
3) Cross-linking the enzyme by addition of a di-aldehyde. The cross-linking agent preferably is one or more of glutaraldehyde, glyoxal, malondialdehyde, succindialdehyde, phthaladehyde, and more preferably is glutaraldehyde. With glutaraldehyde as cross-linking agent especially good results were obtained. Preferably the cross-linking is performed by addition of glutaraldehyde in the presence of ammonium sulphate. Preferably the ammonium sulphate is added first and the mixture stirred for at least 20 seconds before addition of the glutaraldehyde. Preferably the amount of ammonium sulphate at step 3) is from 30 to 95 wt.%, more preferably from 50 to 90 wt.% and even more preferably from 70 to 85 wt.%, based on the weight of the mixture at step 3). Preferably the amount of di-aldehyde at step 3) is from 0.1 to 200mM, more preferably from 1 to 50 mM, even more preferably of from 2 to 30 mM and still even more preferably of from 10 to 21 mM.
4) Preferably the reaction mixture is stirred for at least 1 hour, more preferably from 4 to 30 hours and even more preferably from 10 to 20 hours. The temperature for stirring the reaction mixture is preferably below ambient temperature and more preferably from 1 to 15 degrees Celsius.
5) Preferably the CLEAs are washed. More preferably the CLEAs are washed by addition of water and mixing. Preferably after washing the CLEAs are isolated. The isolation of the CLEAs can suitably be performed by centrifugation at conditions suitable to collect separate the CLEAs from the bulk of the liquid phase, and decanting to remove the bulk of said liquid phase. The washing step can be repeated more than once and preferably is repeated from 2 to 4 times.
6) Preferably the isolated and washed CLEAs are re-suspended in a suitable buffer (preferably a buffer as in step 1) before use. The isolated and washed CLEAs are preferably stored at cool and/or dark conditions.

### Surfactant

The liquid detergency composition according to the invention preferably comprises surfactant and more preferably comprises detersive surfactant. By detersive surfactant is meant that the surfactant provides a detersive (i.e. cleaning effect) to textile fabrics treated as part of a cleaning, preferably a laundering, process.

Preferably the total amount of surfactant present in the liquid detergency composition is from 2 to 85 wt.%, more preferably from 3 to 60 wt.%, even more preferably from 4 to 40 wt.% and still even more preferably from 5 to 35 wt.%.

Preferably the detersive surfactant comprises anionic surfactant, nonionic surfactant or a mixture thereof and more preferably comprises anionic and nonionic surfactants.

The surfactant preferably comprises biosurfactant and more preferably biosurfactant derived from bacteria, fungi and/or other microbes. The surfactant preferably comprises one or more of glycolipid biosurfactant (which preferably is a rhamnolipid or sophorolipid or trehalolipid or a mannosylerythritol lipid (MEL)), cellobiose, peptide based biosurfactants, lipoproteins and lipopeptides e.g. surfactin, fatty acids e.g. corynomucolic acids (preferably with hydrocarbon chain C12-C14), phospholipids (preferred phospholipids are phosphatidylethanolamine produced by Rhodococcus erythropolis grown on n-alkane which results in lowering of interfacial tension between water and hexadecane to less than 1 mN m-1 and CMC of 30 mg L-1 (Kretschner et al., 1982) and spiculisporic acid); polymeric biosurfactants including emulsan, liposan, mannoprotein and polysaccharide-protein complexes. Preferably the biosurfactant comprises a rhamnolipid.

The amount of anionic surfactant or nonionic surfactant or the combination thereof preferably is from 0.5 to 95 wt.%, more preferably from 1 to 50 wt.% and even more preferably from 1.5 to 25 wt.%, based on total weight of surfactant. If a detersive surfactant mixture is used that incorporates both anionic and nonionic surfactants, then preferably the ratio of anionic surfactant to nonionic surfactant is from 10:1 to 1:10.

### Nonionic surfactant

'Nonionic surfactant' is defined as amphiphilic molecules with a molecular weight of less than about 10,000, unless otherwise noted, which are substantially free of any functional groups that exhibit a net charge at the normal wash pH of 6-11.

Any type of nonionic surfactant may be used. Nonionic surfactants preferably are fatty acid alkoxylates and more preferably ethoxylates. Preferred ethoxylates have an alkyl chain of from C₈-C₃₅, more preferably C₁₀-C_{24;} and have preferably 3 to 25, more preferred 5 to 15 ethylene oxide groups. These are commercially available such as under Neodols from Shell (The Hague, The Netherlands); ethylene oxide/propylene oxide block polymers which may have molecular weight from 1,000 to 30,000, for example, Pluronic (trademark) from BASF (Ludwigshafen, Germany); and alkylphenol ethoxylates, for example Triton X-100, available from Dow Chemical (Midland, Mich., USA).

### Anionic surfactant

'Anionic surfactants' are defined as amphiphilic molecules comprising one or more functional groups that exhibit a net anionic charge when in aqueous solution at the normal wash pH of between 6 and 11.

Preferred anionic surfactants are the alkali metal salts of organic sulphur reaction products having in their molecular structure an alkyl radical containing from about 6 to 24 carbon atoms and a radical selected from the group consisting of sulphonic and sulphuric acid ester radicals. More preferred anionic surfactants are the alkali and alkaline earth metal salts of fatty acid carboxylates, fatty alcohol sulphates, preferably primary alkyl sulfates, more preferably they are ethoxylated, for example alkyl ether sulfates; and alkylbenzene sulfonates or mixtures thereof.

### Cationic, amphoteric surfactants and/or zwitterionic surfactants

Preferably the liquid detergency composition according to the invention comprises one or more of cationic, amphoteric surfactants and zwitterionic surfactants.

Preferred cationic surfactants are quaternary ammonium salts of the general formula R₁R₂R₃R₄N⁺ X⁻, for example where R₁ is a C₁₂-C₁₄ alkyl group, R₂ and R₃ are methyl groups, R₄ is a 2-hydroxyethyl group, and X⁻ is a chloride ion. This material is available commercially as Praepagen (Trade Mark) HY from Clariant GmbH, in the form of a 40 wt.% aqueous solution.

Amphoteric surfactants are molecules that contain both acidic and basic groups and will exist as zwitterions at the normal wash pH of between 6 and 11. Preferably the amount of amphoteric or zwitterionic surfactant is from 0.1 to 20 wt.%, more preferably from 0.25 to 15 wt.% and even more preferably from 0.5 to 10 wt.%.

Suitable zwitterionic surfactants are exemplified as those which can be broadly described as derivatives of aliphatic quaternary ammonium, sulfonium and phosphonium compounds with one long chain group having about 8 to about 18 carbon atoms and at least one water solubilizing radical selected from the group consisting of sulfate, sulfonate, carboxylate, phosphate or phosphonate. A general formula for these compounds is:

R₁(R₂)ₓY⁺R₃Z⁻

wherein R₁ contains an alkyl, alkenyl or hydroxyalkyl group with 8 to 18 carbon atoms, from 0 to 10 ethylene-oxy groups or from 0 to 2 glyceryl units; Y is a nitrogen, sulfur or phosphorous atom; R₂ is an alkyl or hydroxyalkyl group with 1 to 3 carbon atoms; x is 1 when Y is a sulfur atom and 2 when Y is a nitrogen or phosphorous atom; R₃ is an alkyl or hydroxyalkyl group with 1 to 5 carbon atoms and Z is a radical selected from the group consisting of sulfate, sulfonate, carboxylate, phosphate or phosphonate.

Preferred amphoteric or zwitterionic surfactants are betaine surfactants. More preferably these are one or more from the following list:
Sulfatobetaines, such as 3-(dodecyldimethylammonium)-1-propane sulfate; and 2-(cocodimethylammonium)-1-ethane sulfate. Sulfobetaines, such as: 3-(dodecyldimethyl-ammonium)-2-hydroxy-1-propane sulfonate; 3-(tetradecyldimethylammonium)-1-propane sulfonate; 3-(C₁₂-C₁₄ alkyl-amidopropyldimethylammonium)-2-hydroxy-1-propane sulfonate; and 3-(cocodimethylammonium)-1-propane sulfonate. Carboxybetaines, such as (dodecyldimethylammonium) acetate (also known as lauryl betaine); (tetradecyldimethylammonium) acetate (also known as myristyl betaine); (cocodimethylammonium) acetate (also known as coconut betaine); (oleyldimethylammonium) acetate (also known as oleyl betaine); (dodecyloxymethyldimethylammonium) acetate; and (cocoamido-propyldimethylammonium) acetate (also known as cocoamido-propyl betaine or CAPB). Sulfoniumbetaines, such as: (dodecyldimethylsulfonium) acetate; and 3-(cocodimethyl-sulfonium)-1-propane sulfonate. Phosphoniumbetaines, such as 4-(trimethylphosphonium)-1-hexadecane sulfonate; 3-(dodecyldimethylphosphonium)-1-propanesulfonate; and 2-(dodecyldimethylphosphonium)-1-ethane sulfate.

The liquid detergency composition according to the present invention preferably comprise one or more of carboxybetaines or sulphobetaines as amphoteric or zwitterionic surfactants and more preferably comprises lauryl betaine.

### Bleaching agent

The liquid detergency composition according to the invention preferably comprises bleaching agent. The bleaching agent component for use herein can be any bleaching agents suitable for use in detergency compositions such as oxygen bleaches as well as others known in the art. The bleaching agent can be activated or non-activated bleaching agent.

Preferably the liquid detergency composition according to the invention comprises oxygen bleaching agent, halogen bleaching agent or a combination thereof.

Preferred oxygen bleaching agents are percarboxylic acid bleaching agents and salts thereof and more preferably one or more of magnesium monoperoxyphthalate hexahydrate, the magnesium salt of meta-chloro perbenzoic acid, 4-nonylamino-4- oxoperoxybutyric acid and diperoxydode-canedioic acid or combinations thereof.

Preferably the halogen bleaching agents is one or more of hypohalite bleaching agents, such as trichloro isocyanuric acid and the sodium and potassium dichloroisocyanurates and N-chloro and N-bromo alkane sulphonamides.

Preferably the bleaching agents are added in an amount of from 0.5 to 10 wt.%, more preferably of from 1 to 5 wt.%.

Hydrogen peroxide releasing agents are preferably used in combination with a bleach activators. Preferably the hydrogen peroxide releasing agents is one or more of tetraacetylethylenediamine (TAED), nonanoyloxybenzene-sulfonate, 3, 5, - trimethylhexanoloxybenzenesulfonate (ISONOBS), pentaacetylglucose (PAG), C8(6-octanamido-caproyl)oxybenzenesulfonate, C9(6-nonamido caproyl) oxybenzenesulfonate and C10(6-decanamido caproyl)oxybenzene sulfonate.

### Builder

Preferably the liquid detergency composition according to the invention comprises builder and more preferably comprises one or more of aluminosilicate materials, silicates, polycarboxylates and fatty acids, materials such as ethylenediamine tetraacetate, metal ion sequestrants such as aminopolyphosphonates. Even more preferably, the liquid detergency composition according to the invention comprises zeolite A, citric acid or a combination thereof.

The amount of builder preferably is from 10 to 80 wt.%, more preferably from 20 to 70 wt.% even more preferably from 30 to 60 wt.%.

### Suds suppressor

Preferably the liquid detergency composition according to the invention comprises suds suppressor and more preferably a silica based suds suppressor, a silicon based suds suppressor or a mixture thereof. Even more preferably the liquid detergency composition according to the invention comprises a mixture of silicone oils and 2-alkylalcanols. The silicones refer to alkylated polysiloxane materials. Silica is preferably used in finely divided forms exemplified by silica aerogels and xerogels and hydrophobic silicas of various types.

Preferably the amount of suds suppressors is from 0.001 to 2 wt.% and more preferably from 0. 01 to 1 wt.%.

### Anti redeposition

Preferably the liquid detergency composition according to the invention comprises one or more anti redeposition agents (also known as a soil suspension agent) of methylcellulose, carboxymethylcellulose and hydroxyethylcellulose, and homo- or co-polymeric polycarboxylic acids or their salts.

Preferably the amount of anti redeposition agent is from 0.5 to 10 wt.%, more preferably from 0.75 to 8 wt.% and even more preferably from 1 to 6 wt.%.

### Soli release agent

Preferably the liquid detergency according to the invention comprises one or more soil release agents and more preferably copolymers or terpolymers of terephthalic acid with ethylene glycol and/or propylene glycol units in various arrangements.

### Other optional ingredients

The liquid detergency composition may comprise other ingredients commonly found in detergent liquids. Preferably the detergency composition according to the invention comprises one or more of hydrotropes, opacifiers, colorants, perfumes, microcapsules of ingredients such as perfume or care additives, softeners, antioxidants, pH control agents and buffers.

### Detergency composition manufacture process

The liquid detergency composition according to the invention can be made by simply mixing the (liquid and solid) components.

Preferably the liquid detergency composition according to the invention is in the form of a unit-dosed packaged liquid detergency composition. Such unit-dosed packages are well-known in the art and typically comprise an at least partly water-dissolvable outer-packaging material, which sufficiently disintegrates to enable release of the unit-dosed packaged contents upon contact with sufficient amount of water. A sufficient amount of water for example is an amount of water typically used in a wash-cycle of a standard automated laundry machine. Preferably, the wash cycle involves the use of 10 to 100 litres of water in combination with 5 to 100 grams of liquid detergency composition.

Preferably the unit-dose package comprises from 5 to 100 grams of liquid detergency composition. Preferably, the unit-dose package comprises and more preferably essentially consists of water-dissolvable packaging material.

Preferably the liquid detergency composition according to the invention is a liquid laundry composition.

The invention will now be further described with reference to the following nonlimiting examples as follows:

### Examples

### Production of CLEAs of Lipex:

1) At room temperature in a 50 mL Falcon tube, Lipex enzyme was made to a final concentration of 0.69 µM with 30 mM MES-NaOH, pH 7.5, 150 mM NaCl, 1 mM CaCl₂.
2) **Activator step:** Tween 80 was added to a final concentration of 19mM and the solution left stirring at room temperature on a magnetic plate at 150 rpm for 5 minutes
3) Ammonium sulfate was added to a final concentration 80% and the solution stirred at 150 rpm for 30 seconds prior to the addition of GA at a final concentration of 5 mM.
4) The CLEA reaction was stirred for 17 hours at 4°C at 150rpm in a clear 50 mL Falcon tube
5) To wash the Lipex CLEAs, 27 ml of dH₂O was added to the sample. The Lipex CLEA was mixed for 5 minutes with the water using a pasteur pipette and centrifuged at 24,444g for 40 minutes at 4°C.
6) The supernatant was decanted and 30 ml of dH₂O was added to the CLEA pellet. The CLEA samples were mixed with the dH₂O using a Pasteur pipette until the CLEA particles were dispersed evenly followed by centrifuged at 24,444 x g for 40 min at 4°C. This was repeated three times in order to wash the CLEA thoroughly.
7) The CLEA pellet was dispersed in 5 ml of 30 mM MES-NaOH, pH 7.5, 150 mM NaCl, 1 mM CaCl₂ or in 5 mL of Unilever formulation and mixed at 150 rpm for 17 hours at 4°C in order to obtain a homogeneous preparation of CLEA particles in suspension.

### Reagents:

Lipex 100L (Novozymes)
Lipex 100L CLEA (synthesised as according to the above procedure from Lipex 100L)
Lipase CLEA (Lipase, *Thermomyces lanuginosa, CLEA,* Sigma cat # 07676) Coronase (Novozymes)
Para-nitrophenyl-Valerate (Sigma cat # N4377)
N-Succinyl-Ala-Ala-Pro-Phe p-nitroanilide (Sigma cat # S7388)

### Protocol:

Liquid detergency compositions were made as set out in Table 1. The samples were directly measured (T0) or incubated for a period of 1 week (T1), 2 weeks (T2), 3 weeks (T3) or 4 weeks (T4) weeks at 37°C. Example 1 (Ex. 1) is an example according to the invention. Comparative A (Comp. A) and Comparative B (Comp. B) are not according to the invention.

**Table 1. Liquid detergency compositions (amounts are based on wt.% unless otherwise indicated).**

| | Ex. 1 | Comp. A | Comp. B |
|---|---|---|---|
| **Enzyme combination (Total 0.125 ml)** | | | |
| Lipex 100L CLEA | + | - | - |
| Lipex 100L | - | + | - |
| Lipase CLEA | - | - | + |
| ¹Coronase™ | + | + | + |

| **Liquid laundry formulation (Total 2.375 ml)** | | | |
|---|---|---|---|
| Ethylamine | 7.0 | 7.0 | 7.0 |
| Triethanolamine | 2.5 | 2.5 | 2.5 |
| Monopropylene Glycol | 11.0 | 11.0 | 11.0 |
| Glycerol | 5.0 | 5.0 | 5.0 |
| Citric Acid | 3.9 | 3.9 | 3.9 |
| Bis-(triazinylamino)-stilbene disulfonic acid derivative | 0.1 | 0.1 | 0.1 |
| Alcohol (C12-C16)poly(7-19)ethoxylates | 4.6 | 4.6 | 4.6 |
| Dodecylbenzene Sulfonic Acid | 8.8 | 8.8 | 8.8 |
| Hydrogenated palm kernel fat | 3.0 | 3.0 | 3.0 |
| 1-hydroxy ethylidene-1,1-diphosphonicacid phosphonic acid | 1.5 | 1.5 | 1.5 |
| Sodium Lauryl Ether Sulphate 70 % | 6.8 | 6.8 | 6.8 |
| Polyethyleneimine,ethoxylated | 3.0 | 3.0 | 3.0 |
| Sodium Sulphite | 0.25 | 0.25 | 0.25 |
| Perfume | 1.4 | 1.4 | 1.4 |
| Water | To balance | To balance | To balance |
| | | | |
| **Total (enzyme combination + liquid laundry formulation)** | 2.5ml | 2.5ml | 2.5ml |

| | | | |
|---|---|---|---|
| ¹The same amount of Coronase™ was used in all samples. | | | |

To measure the residual activities of lipase and protease, each sample (2.5mL aliquot) was first added to 250mL of water to disperse the enzyme CLEA before sampling. Each sample was further diluted in Milli-q water to give a final volume of 1000mL.

The assay was carried out in a standard (96 well) microtiter plate. 20µL of sample, 100 µL of Tris-HCI (50mM, pH 8.5), 60µL water and 20µL of substrate (1mM pNP-valerate in 10% methanol, pH 4.5) were added to give 200µL of final assay volume. The lipase activity was measured by monitoring the release of free p-nitrophenol at 405nm over a 15 minute incubation period at room temperature.

### Lipase activity:

The enzyme assay was carried out in Tris-HCI buffer and after each time point, the residual activity of each sample was determined by measuring hydrolytic enzyme activity of substrates as described above. The residual activity (%) was determined, by setting to the lipase activity at T0 to 100 %. The Lipase activities are shown in Table 2.

**Table 2. Lipase residual activities (%),± indicates the standard deviation.**

| **Time (week)** | **Ex. 1** | **Comp. A** | **Comp. B** |
|---|---|---|---|
| **T0** | 100 | 100 | 100 |
| **T1** | 101.42±0.24 | 81.91±1.08 | 114.76±2.37 |
| **T2** | 80.56±1.96 | 59.42±1.66 | 91.23±4.03 |
| **T3** | 69.43±4.45 | 52.69±1.94 | 87.57±7.47 |
| **T4** | 65.69±1.35 | 41.54±2.26 | 56.70±4.46 |

The results in Table 2 show that after 4 weeks, the Lipex 100L CLEA (Example 1) possessed a residual activity of ≥65% while a substantial drop in residual activities were observed with commercial Lipase CLEA (Comparative B) and Lipex 100L (Comparative A). After 4 weeks (T4) the Lipase CLEA residual activity was approximately 56% and the residual activity of the Lipex 100L (i.e. not in CLEA form) was approximately 42%. From this it can be concluded that cross-linked Lipex possess superior activity and stability when stored in the presence of a protease, even when compared to commercially available Lipase CLEA.

The results of the improved stability of the Lipex 100L CLEA are the more surprising given the initial high activity (> 100%) of the commercial Lipase CLEA in the presence of the protease after 1 week (T1). E.g. the residual activity of the commercial Lipase CLEA drops far more quickly when compared to the Lipex 100L CLEA, which would suggest even greater difference in the residual activity over the life-time of a detergency product, which can be far longer than 4 weeks.

## Claims

1. A liquid detergency composition comprising a protease and a lipase, wherein the lipase comprises a polypeptide having an amino acid sequence which has at least 90 percent sequence identity with the wild-type lipase derived from *Humicola lanuginosa* strain DSM 4109 and, compared to said wild-type lipase, comprises a substitution of an electrically neutral or negatively charged amino acid within 15 Å of E1 or Q249 with a positively charged amino acid, wherein at least part of the lipase is cross-linked enzyme aggregate of the lipase.

2. A liquid detergency composition according to claim 1, wherein the amount of the lipase is from 0.01 to 6 wt.%, preferably from 0.1 to 5 wt.%, more preferably from 0.2 to 4 wt.%, even more preferably from 0.5 to 3 wt.% and still even more preferably from 0.7 to 2.0 wt.%

3. A liquid detergency composition according to claim 1 or claim 2, wherein at least 20 wt.% of the lipase is cross-linked enzyme aggregate of the lipase, preferably at least 40 wt.%, more preferably at least 60 wt.%, even more preferably at least 80 wt.% is cross-linked enzyme aggregate of the lipase, based on the total amount of lipase, and still even more preferably the lipase essentially consists of cross-linked enzyme aggregate of the lipase.

4. A liquid detergency composition according to any one of claims 1 to 3, wherein the amount of protease is from 0.01 to 6 wt.%, preferably from 0.1 to 5 wt.%, more preferably from 0.2 to 4 wt.%, even more preferably from 0.5 to 3 wt.% and still even more preferably from 0.7 to 2.0 wt.%.

5. A liquid detergency composition according to any one of claims 1 to 4, wherein the protease comprises Savinase™, Coronase™, Relase™ or mixtures thereof, and preferably essentially is Relase™.

6. A liquid detergency composition according to any one of claims 1 to 5, comprising further enzymes and preferably one or more amylase, phospholyase, cutinase, cellulase, peroxidase, oxidase, pectate lyase and mannose enzymes.

7. A liquid detergency composition according to any one of claims 1 to 6, wherein the liquid detergency composition is ambient-active.

8. A liquid detergency composition according to any one of claims 1 to 7, comprising a detersive surfactant, preferably anionic surfactant, nonionic surfactant or a mixture thereof and more preferably comprises anionic and nonionic surfactants.

9. A liquid detergency composition according to any one of claims 1 to 8, comprising oxygen bleaching agent, halogen bleaching agent or a combination thereof.

10. A liquid detergency composition according to any one of claims 1 to 9, comprising aluminosilicate materials, silicates, polycarboxylates and fatty acids, materials such as ethylenediamine tetraacetate, metal ion sequestrants such as aminopolyphosphonates or a combination thereof, and more preferably comprises zeolite A, citric acid or a combination thereof.

11. A liquid detergency composition according to any one of claims 1 to 10, comprising suds suppressor and preferably a silica based suds suppressor, a silicon based suds suppressor or a mixture thereof.

12. A liquid detergency composition according to any one of claim 1 to 11, comprising one or more anti redeposition agents of methylcellulose, carboxymethylcellulose and hydroxyethylcellulose, and homo- or co-polymeric polycarboxylic acids or their salts.

13. A liquid detergency composition according to any one of claims 1 to 12, wherein the composition is packaged in the form of a unit-dosed packaged liquid detergency composition.

14. A liquid detergency composition according to any one of claims 1 to 13, wherein the composition is a liquid laundry composition.

## Patentansprüche

1. Flüssige Waschmittelzusammensetzung, umfassend eine Protease und eine Lipase, wobei die Lipase ein Polypeptid umfasst, das eine Aminosäuresequenz aufweist, welche mindestens 90 Prozent Sequenzidentität mit der Wildtyp-Lipase, die von dem Stamm *Humicola lanuginosa* DSM 4109 stammt, aufweist und welche, verglichen mit besagter Wildtyp-Lipase, eine Substitution einer elektrisch neutralen oder negativ geladenen Aminosäure innerhalb von 15 Ä von E1 oder Q249 mit einer positiv geladenen Aminosäure umfasst, wobei mindestens ein Teil der Lipase quervernetztes Enzymaggregat der Lipase ist.

2. Flüssige Waschmittelzusammensetzung nach Anspruch 1, wobei die Menge der Lipase von 0,01 bis 6 Gew.-%, bevorzugt von 0,1 bis 5 Gew.-%, bevorzugter von 0,2 bis 4 Gew.-%, noch bevorzugter von 0,5 bis 3 Gew.-% und sogar noch bevorzugter von 0,7 bis 2,0 Gew.-%, beträgt.

3. Flüssige Waschmittelzusammensetzung nach Anspruch 1 oder Anspruch 2, wobei mindestens 20 Gew.-% der Lipase quervernetztes Enzymaggregat der Lipase ist, vorzugsweise mindestens 40 Gew.-%, bevorzugter mindestens 60 Gew.-%, noch bevorzugter mindestens 80 Gew.-% quervernetztes Enzymaggregat der Lipase ist, bezogen auf die Gesamtmenge an Lipase, und die Lipase sogar noch bevorzugter im Wesentlichen aus quervernetztem Enzymaggregat der Lipase besteht.

4. Flüssige Waschmittelzusammensetzung nach irgendeinem der Ansprüche 1 bis 3, wobei die Menge an Protease von 0,01 bis 6 Gew.-%, bevorzugt von 0,1 bis 5 Gew.-%, bevorzugter von 0,2 bis 4 Gew.-%, noch bevorzugter von 0,5 bis 3 Gew.-% und sogar noch bevorzugter von 0,7 bis 2,0 Gew.-%, beträgt.

5. Flüssige Waschmittelzusammensetzung nach irgendeinem der Ansprüche 1 bis 4, wobei die Protease Savinase™, Coronase™, Relase™ oder Mischungen davon umfasst und vorzugsweise im Wesentlichen Relase™ ist.

6. Flüssige Waschmittelzusammensetzung nach irgendeinem der Ansprüche 1 bis 5, umfassend ferner Enzyme und vorzugsweise ein oder mehrere Amylase, Phosphorylase, Cutinase, Cellulase, Peroxidase, Oxidase, Pectatlyase und Mannoseenzyme.

7. Flüssige Waschmittelzusammensetzung nach irgendeinem der Ansprüche 1 bis 6, wobei die flüssige Waschmittelzusammensetzung umgebungsaktiv ist.

8. Flüssige Waschmittelzusammensetzung nach irgendeinem der Ansprüche 1 bis 7, umfassend ein reinigendes Tensid, vorzugsweise anionisches Tensid, nichtionisches Tensid oder eine Mischung davon, und bevorzugter umfassend anionische und nichtionische Tenside.

9. Flüssige Waschmittelzusammensetzung nach irgendeinem der Ansprüche 1 bis 8, umfassend ein sauerstoffhaltiges Bleichmittel, halogenhaltiges Bleichmittel oder eine Kombination davon.

10. Flüssige Waschmittelzusammensetzung nach irgendeinem der Ansprüche 1 bis 9, umfassend Aluminosilikatmaterialien, Silikate, Polycarboxylate und Fettsäuren, Materialien wie z.B. Ethylendiamintetraacetat, Metallionenchelatbildner wie z.B. Aminopolyphosphonate oder eine Kombination davon und bevorzugter umfassend Zeolith A, Zitronensäure oder eine Kombination davon.

11. Flüssige Waschmittelzusammensetzung nach irgendeinem der Ansprüche 1 bis 10, umfassend Schaumunterdrücker und vorzugsweise einen kieselsäurebasierten Schaumunterdrücker, einen siliziumbasierten Schaumunterdrücker oder eine Mischung davon.

12. Flüssige Waschmittelzusammensetzung nach irgendeinem der Ansprüche 1 bis 11, umfassend ein oder mehrere Anti-Wiederablagerungsmittel von Methylcellulose, Carboxymethylcellulose und Hydroxyethylcellulose und homo- oder copolymere Polycarboxylsäuren oder deren Salze.

13. Flüssige Waschmittelzusammensetzung nach irgendeinem der Ansprüche 1 bis 12, wobei die Zusammensetzung in der Form einer einzeldosisverpackten flüssigen Waschmittelzusammensetzung verpackt ist.

14. Flüssige Waschmittelzusammensetzung nach irgendeinem der Ansprüche 1 bis 13, wobei die Zusammensetzung eine flüssige Reinigungszusammensetzung ist.

## Revendications

1. Composition de détergence liquide comprenant une protéase et une lipase, dans laquelle la lipase comprend un polypeptide ayant une séquence d'acide aminé qui présente au moins 90 pourcent d'identité de séquence avec la lipase de type sauvage dérivée de la souche DSM 4109 *Humicola lanuginosa* et, comparée à ladite lipase de type sauvage, comprend une substitution d'un acide aminé électriquement neutre ou négativement chargé avec 15 Å de E1 ou Q249 avec un acide aminé positivement chargé, dans laquelle au moins une partie de la lipase est un agrégat d'enzyme réticulé de la lipase.

2. Composition de détergence liquide selon la revendication 1, dans laquelle la quantité de la lipase est de 0,01 à 6 % en masse, de préférence de 0,1 à 5 % en masse, encore mieux de 0,2 à 4 % en masse, bien mieux encore de 0,5 à 3 % en masse et particulièrement de préférence de 0,7 à 2,0 % en masse.

3. Composition de détergence liquide selon la revendication 1 ou la revendication 2, dans laquelle au moins 20 % en masse de la lipase sont un agrégat d'enzyme réticulée de la lipase, de préférence au moins 40 % en masse, encore mieux au moins 60 % en masse, bien mieux encore au moins 80 % en masse sont un agrégat d'enzyme réticulée de la lipase, rapporté à la quantité totale de lipase, et particulièrement de préférence la lipase est essentiellement constituée d'agrégat d'enzyme réticulée de la lipase.

4. Composition de détergence liquide selon l'une quelconque des revendications 1 à 3, dans laquelle la quantité de protéase est de 0,01 à 6 % en masse, de préférence de 0,1 à 5 % en masse, encore mieux de 0,2 à 4 % en masse, bien mieux encore de 0,5 à 3 % en masse et particulièrement de préférence de 0,7 à 2,0 % en masse.

5. Composition de détergence liquide selon l'une quelconque des revendications 1 à 4, dans laquelle la protéase comprend Savinase™, Coronase™, Relase™ ou des mélanges de celles-ci, et de préférence est essentiellement Relase™.

6. Composition de détergence liquide selon l'une quelconque des revendications 1 à 5, comprenant de plus des enzymes et de préférence une ou plusieurs d'enzymes d'amylase, phospholyase, cutinase, cellulase, peroxydase, oxydase, pectate lyase et mannose.

7. Composition de détergence liquide selon l'une quelconque des revendications 1 à 6, dans laquelle la composition de détergence liquide est active dans les conditions ambiantes.

8. Composition de détergence liquide selon l'une quelconque des revendications 1 à 7, comprenant un tensioactif détersif, de préférence un tensioactif anionique, un tensioactif non-ionique ou un mélange de ceux-ci et comprend encore mieux des tensioactifs anioniques et non-ioniques.

9. Composition de détergence liquide selon l'une quelconque des revendications 1 à 8, comprenant un agent blanchissant d'oxygène, un agent blanchissant d'halogène ou une combinaison de ceux-ci.

10. Composition de détergence liquide selon l'une quelconque des revendications 1 à 9, comprenant des matériaux d'aluminosilicate, des silicates, des polycarboxylates et des acides gras, des matériaux tels que le tétraacétate d'éthylènediamine, des séquestrants d'ions de métaux tels que des aminopolyphosphonates ou une combinaison de ceux-ci, et comprend encore mieux de la zéolite A, de l'acide citrique ou une combinaison de ceux-ci.

11. Composition de détergence liquide selon l'une quelconque des revendications 1 à 10, comprenant un suppresseur de mousse et de préférence un suppresseur de mousse à base de silice, un suppresseur de mousse à base de silicium ou un mélange de ceux-ci.

12. Composition de détergence liquide selon l'une quelconque des revendications 1 à 11, comprenant un ou plusieurs agents anti-redéposition de méthylcellulose, carboxyméthylcellulose et hydroxyéthylcellulose, et des acides polycarboxyliques homo- ou copolymères ou leurs sels.

13. Composition de détergence liquide selon l'une quelconque des revendications 1 à 12, dans laquelle la composition est emballée dans la forme d'une composition de détergence liquide emballée en dose unitaire.

14. Composition de détergence liquide selon l'une quelconque des revendications 1 à 13, dans laquelle la composition est une composition de lessive liquide.
